# EUROPEAN PATENT APPLICATION

(11) **EP 1 358 877 A1**
(43) Date of publication of application: **05.11.2003**
(21) Application number: 01272331.8
(22) Date of filing: 26.12.2001
(51) Int. Cl.: A61K 31/198, A61P 1/02, A61P 31/04

(54) **COMPOSITIONS FOR ORAL USE**

(30) Priority: 26.12.2000 JP 2000394547
(71) Applicant: Koga, Keiko, Fukuoka-shi, Fukuoka 812-0017 (JP); Kyushu TLO Company, Limited, Fukuoka-shi, Fukuoka 812-8581 (JP); TAISHO PHARMACEUTICAL CO., LTD, Tokyo 171-0033 (JP)
(72) Inventor: NAKANO, Yoshio, Fukuoka-shi, Fukuoka 810-0016 (JP); YOSHIMURA, Mamiko, Nagasaki-shi, Nagasaki 852-8105 (JP); KOGA, Toshihiko, deceased (JP)
(74) Representative: HOFFMANN - EITLE
(86) International application number: JP0111457
(87) International publication number: WO02051404

(57) **Abstract**

An oral composition comprising trifluoromethionine as an effective component is provided as a useful oral composition for preventing and/or treating intraoral diseases such as bad breath, periodontal disease, alveolar pyorrhea, etc., which is safe even if daily used.

## Description

### TECHNICAL FIELD

The present invention relates to a composition for preventing and/or treating intraoral diseases such as bad breath, alveolar pyorrhea, periodontal disease, etc., and more particularly to a composition comprising trifluoromethionine as an effective component for preventing and/or treating intraoral diseases such as bad breath, alveolar pyorrhea, periodontal disease, etc.

### BACKGROUND ART

Recently, there has been a growing interest in prevention and treatment of intraoral diseases such as bad breath, alveolar pyorrhea, periodontal disease, etc.

Most important causative substances of bad breath are so-called volatile sulfides such as hydrogen sulfide, methylmercaptan and dimethyl sulfide, among which methylmercaptan is known to be produced by many causative organisms of periodontisis and thus has been watched with interest as an indicator of periodontisis and also as one of pathogenic factors of periodontisis. The methylmercaptan is produced from methionine by L-methionine-α-deamino-γ-mercaptomethane-lyase(METase) as an enzyme contained in bacteria.

Among causative organisms of periodontal disease, well known organisms capable of efficiently producing methylmercaptan include bacteria of genus *Porphyromonas* such as *Porphyromonas gingivalis,* etc., bacteria of genus *Fusobacterium* such as *Fusobacterium nucleatum*, etc.

Thus, it can be presumed that prevention and treatment of the above-mentioned intraoral diseases can be effectively attained by inhibiting propagation of these bacteria, and the ordinary antibacterial active substances have a possibility to kill the bacteria, thereby effectively attaining prevention and treatment of bad breath, etc. However, the ordinary antibacterial active substances can kill even intraoral indigenous bacteria free from pathogenicity, and thus are not recommendable from the viewpoint of preventing fixation of harmful exogenetic bacteria.

Thus, it has been presumed useful to find substances having a bactericidal effect or a growth inhibiting effect selectively on the methylmercaptan-producing organisms, which have been presumed to cause the above-mentioned intraoral diseases.

### DISCLOSURE OF THE INVENTION

An object of the present invention is to provide an oral composition comprising a substance having a bactericidal effect or growth-inhibiting effect selectively on the above-mentioned causative organisms of intraoral diseases as an effective component.

In preparing METase-deficient strains of the above-mentioned intraoral organisms, the present inventors have found that the METase-deficient strains have no ability of producing methylmercaptan from methionine, showing that only METase takes part in the synthesis of methylmercaptan. METase is possessed by substantially all the organisms, but not by mammals including human beings. Thus, it can be presumed that a preventive and treating preparation of high safety can be provided for intraoral diseases by using a substance having a growth-inhibiting effect on the causative organisms of intraoral diseases, aiming at the METase as a target.

As a result of investigations of growth-inhibiting effects on methylmercaptan-producing bacteria, using trifluoromethionine as a methionine derivative from the above-mentioned viewpoint, the present inventors have found that trifluoromethionine has a remarkable growth-inhibiting effect.

That is, the present invention provides an oral composition, which comprises trifluoromethionine as an effective component.

According to a preferable mode of the present invention, the present oral composition is used in prevention and/or treatment of bad breath, alveolar pyorrhea, and/or periodontal disease.

### BRIEF DESCRIPTION OF THE DRAWINGS

Fig. 1 is a diagram showing results of antibacterial effect tests of trifluoromethionine as an effective component of the present oral composition on the strain *Porphyromonas gingivalis* W83.
Fig. 2 is a diagram showing results of antibacterial effect tests of trifluoromethionine as an effective component of the present oral composition on the strain *Porphyromonas gingivalis* ATCC33277.
Fig. 3 is a diagram showing results of antibacterial effect tests of trifluoromethionine as an effective component of the present oral composition on the strain *Porphyromonas gingivalis* M1217.
Fig. 4 is a diagram showing results of antibacterial effect tests of trifluoromethionine as an effective component of the present oral composition on the strain *Fusobacterium nucleatum* ATCC10953.
Fig. 5 is a diagram showing results of antibacterial effect tests of trifluoromethionine as an effective component of the present oral composition on the strain *Actinobacillus actinomycetemcomitans* Y4.
Fig. 6 is a diagram showing results of antibacterial effect tests of trifluoromethionine as an effective component of the present oral composition on the strain *Escherichia coli* BL21.

### BEST MODE FOR CARRYING OUT THE INVENTION

The present invention will be described in detail below:

The present oral composition comprises trifluoromethionine as an effective component. Trifluoromethionine per se is a well known compound and can be prepared according to a process, for example, as disclosed in H. Duewel, E. Daub, V. Robinson, and J.F. Honek: Biochemistry, 36:3404-3416(1997).

The form of the present oral composition is not particularly limited, and any form in common use for oral application is acceptable. That is, the forms of the present oral composition include, for example, troche, tooth powder, tooth paste, tooth washes (dental linses), mouth washes, gargles, cream, gel, pasta, spray, foam, etc. and further include edible forms such as candy, chewing gum, etc.

To make the present oral composition into the above-mentioned forms, the present oral composition can contain carriers and additives in common use for oral compositions besides the above-mentioned effective component.

Carriers or additives for use in the present oral composition include, for example, an abrasive, a demulcent, a moistening agent, a thickening agent, a surfactant, a perfume, a sweetening agent, an antiseptic, a preservative, a coloring agent, a pH controlling agent, and other effective compounds, and also a solvent, etc.

The abrasive includes, for example, silica-bases abrasives such as precipitated silica, silica gel, aluminosilicate, zirconosilicate, etc., calcium secondary phosphate dihydrate and anhydrous calcium secondary phosphate, calcium hydrogen phosphate, insoluble sodium metaphosphate, potassium metaphosphate, calcium pyrophosphate, magnesium tertiary phosphate, calcium carbonate, aluminum hydroxide, alumina, magnesium carbonate, zeolite, silicic acid anhydride, silicic acid hydrate, aluminum silicate, zirconium silicate, bentonite, zeolite, aluminum oxide, aluminum hydroxide, synthetic resin-based abrasives, and their mixtures.

The demulcent includes, for example, sodium carboxymethylcellulose, methylcellulose, hydroxyethylcellulose, alginates, carragheenan, gum arabic, polyvinyl alcohol, xanthane gum, gum tragacanth, starch, sodium polyacrylate, Carbopol, gum guar, gum arabic, gelatin, etc.

The moistening agent includes, for example, polyethylene glycol, propylene glycol, sorbitol, glycerin, naltitol, xylitol, etc.

The thickening agent includes, for example, glycerin, sorbitol, propylene glycol, polyethylene glycol, xylitol, maltitol, lactitol etc.

The surfactant is used as a forming agent or a stabilizer for lipophilic substances. The surfactant includes, for example, anionic, nonionic, cationic and amphoteric surfactants, and specifically includes sodium alkylsulfate, alkylphosphoric acid ester, sodium alkylbenzenesulfonate, sodium N-acylsarcosinate, N-acylglutamates, polyoxyethylene hardened castor oil, polyoxyethylene-polypropylene block copolymer (Pluronic type surfactant), sucrose fatty acid esters, alkylglycosides, sorbitan fatty acid esters, polyoxyethylene sorbitan fatty acid esters, alkyldimethylamine oxide, alkylbetaine, polyoxyethylene hardened castor oil, alkylolamide polyglycerin fatty acid esters, etc.

The perfume includes, for example, natural perfumes such as spearmint oil, peppermint oil, wintergreen oil, sassafras oil, clove oil, sage oil, eucalyptus oil, majorana oil, cinnamon oil, thyme oil, mentha oil, orange oil, wintergreen oil, lemon oil, orange oil, etc, and synthetic perfumes such as 1-menthol, anethole, carvone, eugenol, limonene, thymol, methyl salicylate, etc.

The sweetening agent includes, for example, saccharin, saccharin sodium, dextrose, xylitol, stevioside, neohesperidyldihydrochalcone, perillartine, p-methoxycinnamic aldehyde, glycyrrhizinates, aspartame (aspartylphenylalanine methyl ester), Stevia extract etc.

The antiseptic includes, for example, benzoic acid, sodium benzoate, parahydroxybenzoic acid ester, salicylic acid, sodium salicylate, sorbic acid, isopropylmethylphenol, etc.

The preservative includes, for example, methylparaben, propylparaben, benzoate, sodium benzoate, paraoxybenzoic acid ester, titanium dioxide, etc.

The bactericide includes, for example, chlorohexidines, quaternary ammonium salts, triclosan, alkyldiaminoethylglycin hydrochloride, etc. Water soluble fluoride includes, for example, sodium fluoride, sodium monofluorophosphate, etc.

The coloring agent includes, for example, Blue No. 1, Yellow No. 4, titanium dioxide, etc.

The pH controlling agent includes, for example, citric acid and its salts, phosphoric acid and its salts, malic acid and its salts, gluconic acid and its salts, maleic acid and its salts, aspartic acid and its salts, succinic acid and its salts, glucuronic acid and its salts, fumaric acid and its salts, glutamic acid and its salts, adipic acid and its salts, hydrochloric acid, alkali metal hydroxides, etc.

Other effective components for use in the present invention include, for example, so far well known antibacterial agents, bad breath-preventing agents, etc., and specifically chlorophyll compounds, sodium chloride, vitamin C, vitamin E, nicotinic acid ester, allantoin chlorohydroxyaluminum, azulene, lysozyme chloride, hinokitiol, glycyrrhizic acid, dipotassium glycyrrhizinate, protease, fluorides such as sodium fluoride, potassium fluoride, ammonium fluoride, stannous fluoride, sodium monofluorophosphate, etc., water-soluble phosphoric acid compounds such as potassium salt, sodium salt, etc. of orthophosphoric acid, allantoin chlorohydroxyaluminum, hinokitiol, ascorbic acid, lysozyme chloride, glycyrrhizic acid and its salts, sodium chloride, tranexamic acid, ε-aminocaproic acid, dl-tocopherol acetate, azulene, glycyrrhetic acid, sodium copper chlorophyllin, copper gluconate, aluminum lactate, strontium chloride, potassium nitrate, berberine, hydroxamic acid and its derivatives, sodium tripolyphosphate, zeolite, dextranase, mutanase, amylase, methoxyethylene, maleic anhydride copolymer, polyvinylpyrrolidone, epidihydrocholesterin, dihydrocholesterol, benzethonium chloride, trichlorocarbanilide, zinc citrate, and crude drug extracts such as extracts of Japanese angelica roots, phellodendron barks, clove, rosemary, scutellaria roots, safflower etc.

It is preferable in view of the gist of the present invention to use the above-mentioned other effective components in such an amount as not to kill the intraoral indigenous bacteria and as admitted to be safe even in ordinary common use.

The solvent includes, for example, water, ethanol, etc.

The present oral composition can be prepared by mixing trifluoromethionine as an effective component or a trifluoromethionine-producing compound with the above-mentioned carriers and additives by a well known method.

Kinds and mixing proportions of the above-mentioned carriers and additives to be contained in the present oral composition are not particularly limited, but can be selected by those skilled in the art as desired in view of the individual forms of the above-mentioned oral composition.

For example, in the case of a liquid composition such as tooth wash, mouth wash, etc., approximately 0.01-10% of a surfactant, 1-30% of a moistening agent and 50-95% of a solvent can be contained. In the case of a paste composition such as tooth paste, cream, etc., approximately 10-75% of an abrasive, 0.5-10% of a demulcent, and 10-85% of a moistening agent and water can be contained. A perfume, a sweetening agent, etc. can be contained usually in a range of approximately 0.01-5%. A pH of the present oral composition is preferably approximately 4-11, particularly 5-10.

Content of trifluoromethionine as an effective component in the present oral composition is not particularly limited, but usually approximately 0.01-1% by weight, preferably 0.05-0.5% by weight, more preferably 0.1-0.2% by weight, on the basis of the composition.

It is presumed that trifluoromethionine as an effective component of the present invention is converted to trifluoromethylmercaptan by METase of intraoral bacteria and propagation of intraoral bacteria is controlled by the trifluoromethylmercaptan. Trifluoromethylmercaptan is in a gaseous state at the ordinary temperatures, and thus is not retained in the body, assuring a high safety. It should be understood that the present invention is not restricted by any specific theory.

### Examples

The present invention will be described in detail below, referring to Examples, which should be understood to be not restrictive of the present invention.

### Example 1

Antibacterial activity of trifluoromethionine on causative organism of periodontal disease

### (Preparation Example)

Trifluoromethionine (TFM) prepared by Wako Pure Chemicals Industries, Ltd. according to a method disclosed in H. Duewel, E. Daub, V. Robinson and J.F.Honek, Biochemistry, 36:3404-3416(1997) was used. In all of the following Examples, the same TFM was used.

### (Antibacterial Test)

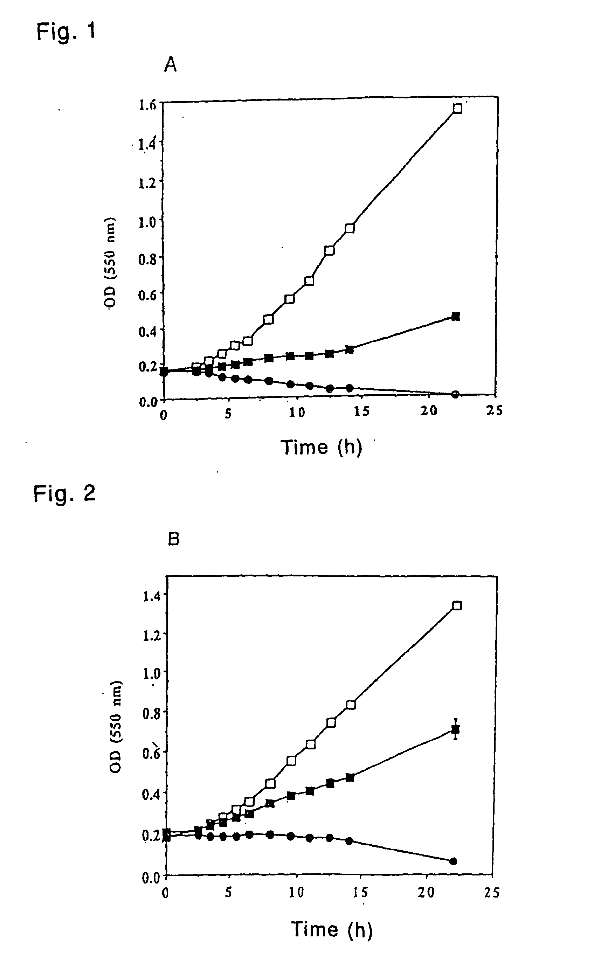

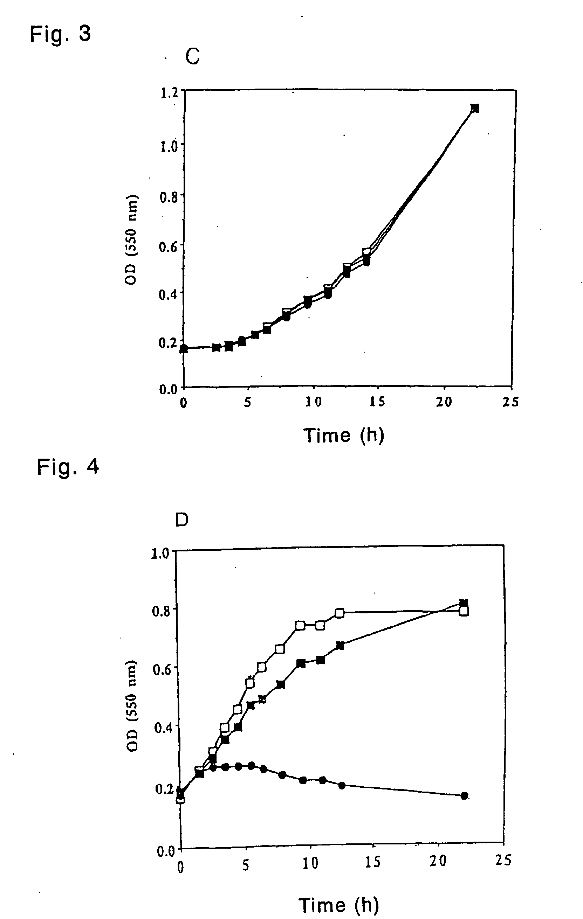

3 ml of GAM broth (Nissui Medical) containing 5 µg/ml of hemin and 1 µg/ml of menadione was separately incubated with one platinum loop each of subcultures of strains *Porphyromonas gingivalis* W83, ATCC33277 and M1217, and strain Fusobacterium nucleatum ATCC10953, followed by incubation at 37°C under anaerobic conditions (10% CO₂, 10% H₂, 80% N₂) overnight. The same fresh medium as above was inoculated with the resulting culture in such an amount that an absorbance at 550 nm of the inoculated medium could be about 0.15, followed by incubation under the same condition as above. Furthermore, the above-mentioned strains were incubated in the above-mentioned medium further containing 0.1 mM or 1.0 mM trifluoromethionine. Absorbance at 550 nm of the individual cultures was measured with time to evaluate propagation of bacteria through the turbidity of the medium. Results are shown in Fig. 1 to 4 (A to D), where the results of using the trifluoromethionine-free medium are shown by □ curve, and those of using media containing 0.1 mM and 1.0 mM trifluoromethionine, respectively, are shown by ■ and ● curves.

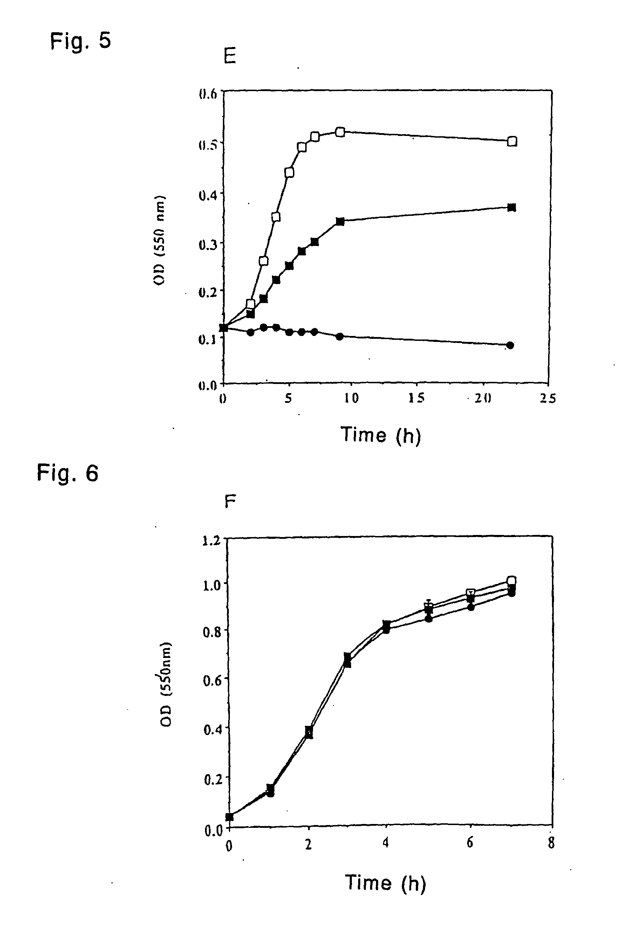

Strain, *Actinobacillus actinomycetemcomitans* Y4, an intraoral bacteria, was incubated in a THY broth (containing 30 g of trypticase soy broth [BBL Microbiology Systems, Cockeysville, Md.] and 10 g of yeast extract [Difco] per liter) in the same manner as above except that the incubation was conducted in the presence of 5% CO₂. Likewise, the strain was incubated in the same medium further containing 0.1 mM or 1.0 mM trifluoromethionine to evaluate propagation of the bacteria. Furthermore, strain *Escherichia coli* BL 21 was incubated in 2xTY broth (containing 16 g of bactotrypton (Difco), 10 g of yeast extract (Difco) and 5 g of sodium chloride per liter) in the same manner and also the strain was likewise incubated in the same medium further containing 0.1 mM or 1.0 mM trifluoromethionine to evaluate propagation of the bacteria. The results are shown in Fig. 5 and 6 (E and F), respectively, where the results of using the trifluoromethionine-free medium are shown by curve □, and the results of using the media containing 0.1 mM and 1.0 mM trifluoromethionine, respectively, are shown by curves ■ and ●.

As is evident from the results shown in Figs. 1 to 6, trifluoromethionine failed to show a significant antibacterial effect on enterobacteria *Escherichia coli,* but showed remarkable antibacterial effects on intraoral bacteria *Porphyromonas gingivalis, Fusobacterium nucleatum* and *Actinobacillus actinomycetemcomitans.* Thus, trifluoromethionine as an effective component of the present oral composition can specifically inhibit propagation of intraoral bacteria including methylmercaptan-producing ones, and are effective for prevention and treatment of intraoral diseases such as bad breath, periodontal disease, etc. and furthermore it seems that trifluoromethionine is incapable of killing intraoral nonpathogenic indigenous bacteria.

### Example 2

Antibacterial activity of trifluoromethionine on causative bacteria of periodontal disease (determination of minimum growth-inhibiting concentration)

3 ml of GAM broth (Nissui Medical) containing 5 µg/ml of hemin and 1 µg/ml of menadione was separately inoculated with one platinum loop each of subcultures of causative bacteria of periodontal disease, strains *Porphyromonas gingivalis* W83, W50, ATCC49417 and ATCC33277, strain *Actinobacillus actinomycetemcomitans* Y4, and strains *Fusobacterium nucleatum* ATCC10953 and ATCC25586, followed by incubation at 37°C under anaerobic conditions (10% CO₂, 10% H₂ and 80% N₂) overnight. Then, the resulting cultures were diluted to 10⁻⁸,10⁻⁶, 10⁻⁴ and 10⁻³ to make inoculating bacterial solutions, respectively.

Agar media containing 400, 200, 100, 50, 25, 10 and 5 µg/ml of TFM, respectively, were prepared and streaked, about 2cm long, with a platinum loop each of the above-prepared inoculating bacterial solutions, followed by incubation under the same anaerobic conditions as above at 37°C for 48-72 hours to evaluate growth of the bacteria. Minimum concentration at which the growth was clearly inhibited by visual observation was made a minimum growth-inhibiting concentration.

*Streptococcus sobrinus* MT8246, *Streptococcus sanuis* ST160, *Streptococcus mutans* Xc and *Streptococcus salivarius HHT* were likewise incubated in Brain Heart Infusion (Difco) in the same manner as above except that the incubation was conducted in the presence of 5% CO₂ to evaluate the growth.

Minimum growth-inhibiting concentrations of TFM determined above for the individual bacteria are summarized in the following Table.

**Table 1**

| Strain | | Minimum growth-inhibiting concentration |
|---|---|---|
| *P. gingivails* | W83 | 5 µg/ml |
| | W50 | 5 µg/ml |
| | ATCC 49417 | 5 µg/ml |
| | ATCC 33277 | 5 µg/ml |
| *A. actinomycetemcomitans* | Y4 | 10 µg/ml |
| *F. nucleatuim* | ATCC 10953 | 25 µg/ml |
| | ATCC 25586 | 25 µg/ml |
| *S. sobrinus* | MT8246 | >400 µg/ml |
| *S. sanguis* | ST160 | >400 µg/ml |
| *S. mutans* | Xc | >400 µg/ml |
| *S. salivarius* | HHT | >400 µg/ml |

It can be seen from the foregoing results that TFM shows selective antibacterial activity on the causative bacteria of periodontal disease.

### Example 3

Determination of minimum growth-inhibiting concentration of trifluoromethionine on standard strains:

Minimum growth-inhibiting concentrations of trifluoromethionine and other antibacterial drugs on standard strains were determined by an agar plate dilution method according to the standard method of Japanese Society of Chemotherapy.

### (Preparation of drug plates)

Drug solutions at a concentration 10 times as high as the maximum applicable concentration were prepared as original solutions of test drugs given in Table 2 (as to solvents used for this purpose, reference should be made also to Table 2). Serial two-fold dilution series of the original solutions were prepared at desired stages, and 1 ml each of the diluted drug solutions was added to 9 ml of media given in Table 3 to prepare drug plates. Concentration range of the individual drugs used are as follows: trifluoromethionine: 400-0.025 µg/ml (15 stages), tetracycline: 100-0.025 µg/ml (13 stages), and clarithromycin: 100-0.025 µg/ml (13 stages).

### (Preparation of inoculating bacterial solution, incubation of bacteria and determination of growth)

1) Bacterial solutions stocked at -80°C were inoculated on agar media (as to media used, reference should be made to Table 3), and then incubated at 37°C overnight. When desired, subculturing was conducted once more on the same agar media (by overnight incubation at 37°C).
2) Colonies growing on the agar media were sampled by a platinum loop, suspended in liquid media (reference should be made to Table 3) and statically incubated at 37°C for 18 hours (preculturing).
3) Preculturing was continued up to McFarland 0.5 (McFarland 0.5 = about 10⁸ CFU/ml), and then the culture broths were diluted with Muellar Hinton Broth to make 10⁶ CFU/ml. 5 µl each of the diluted solutions were inoculated on the above-mentioned drug plates by an inoculator Microplanter.
   "McFarland" is a turbidity indicator in common use in the microbiological field, and an *E. coli* ATCC 25922 suspension at the same turbidity as McFarland 0.5 shows 1-2×10⁸ CFU/ml. Thus, bacterial solutions at about 10⁸ CFU/ml can be easily prepared by use of McFarland 0.5. Bacterial solutions at such a turbidity are commercially available, but can be easily prepared. McFarland 0.5 can be obtained by adding 0.5 ml of an aqueous 0.048 mol/1 barium chloride solution to 99.5 ml of 1% v/v (0.18 mol/l) sulfuric acid.
4) The individual bacteria were incubated at 37°C for 18-20 hours and then the growth was determined in the same manner as in Example 2 to obtain minimum growth-inhibiting concentrations.
   Minimum growth-inhibiting concentrations (MIC) determined above are shown in Table 4.

**Table 2**

| Drug | Solvent for original solution | Solvent for dilution |
|---|---|---|
| Trifluoromethionine | Distilled water | Distilled water |
| Tetracycline | Distilled water | Distilled water |
| Clarithromycin | Methanol | 0.1M, pH6.5 phosphate buffer |

**Table 3**

| Strain used | Agar medium for preculturing | Liquid medium for preculturing | Medium for determination of MIC |
|---|---|---|---|
| *Staphylococcus aureus* | HIA | MHB | MHA |
| *Staphylococcus epidermidis* | HIA | MHB | MHA |
| *Streptococcus pneumoniae* | sheep blood agar medium | MHB+5%LHB | MHA+5%Defibrinated horse blood |
| *Streptococcus pyogenes* | sheep blood agar medium | MHB+5%LHB | MHA+5%Defibrinated horse blood |
| *Streptococcus agalactiae* | sheep blood agar medium | MHB-5%LHB | MHA+5%Defibrinated horse blood |
| *Enterococcus faecalis* | HIA | MHB | MHA |
| *Enterococcus faecium* | HIA | MHB | MHA |
| *Micrococcus luteus* | HIA | MHB | MHA |
| *Bacillus subtilis* | HIA | MHB | MHA |
| *Moraxella (Branhamella) catarrhalis* | sheep blood agar medium agar medium | MHB+5%LHB | MHA+5%Defibrinated horse blood horse blood |
| *Pseudomonas aeruginosa* | HIA | MHB | MHA |
| *Escherichia coli* | HIA | MHB | MHA |
| *Haemophilus influenzae* | chocolate agar | MHB+5%Fildes MHB+5%Fildes | 5% horse chocolate |
| *Serratia marcescens* | HIA | MHB | MHA |
| *Klebsiella pneumoniae* | HIA | MHB | MHA |
| HIA: Heart Infusion Agar, MHB: Muellar Hinton Broth, | | | |
| LHB: Lysed Horse Blood, MHA: Muellar Hinton Agar | | | |

**Table 4**

| | Species | Strain | MIC (µg/ml) | | |
|---|---|---|---|---|---|
| | | | TFM | CAM | TC |
| Gram-positive cocci | *Staphylococcus aureus* | ATCC6538P | >400 | 0.20 | 0.20 |
| | | ATCC29247 | >400 | 50 | 0.78 |
| | *Staphylococcus epidermidis* | ATCC12228 | >400 | 0.20 | 50 |
| | | ATCC29886 | >400 | 0.39 | 0.39 |
| | *Enterococcus faecalis* | ATCC29247 | >400 | 1.56 | 25 |
| | *Enterococcus faecium* | ATCC19434 | >400 | 1.56 | 0.20 |
| | *Micrococcus luteus* | ATCC9341 | >400 | 0.05 | 0.78 |
| | *Streptococcus pneumonias* | ATCC49619 | >400 | 0.05 | 0.10 |
| | | ATCC6301 | >400 | 0.05 | 0.10 |
| | | ATCC6302 | >400 | 0.10 | 0.20 |
| | | ATCC6303 | >400 | 0.05 | 0.10 |
| | | ATCC6306 | >400 | 0.10 | 0.20 |
| | *Streptococcus pyoganes* | ATCC12385 | >400 | 0.10 | 0.20 |
| | | ATCC12353 | >400 | 0.10 | 0.20 |
| | | ATCC12344 | >400 | 0.05 | 0.20 |
| | *Streptococcus agalactiae* | ATCC13813 | >400 | 0.05 | 0.20 |
| Gram-positive bacillus | *Bacillus subtilis* | ATCC6633 | >400 | 0.10 | 0.20 |
| Gram-negative cocci | *Moraxella (Branhamella) catarrhalis* | ATCC25238 | 100 | 0.10 | 0.20 |
| | | ATCC25240 | 25 | 0.20 | 0.20 |
| | | ATCC43617 | 100 | 0.20 | 0.20 |
| Gram-negative bacilli | *Pseudomonas aeruginosa* | ATCC27853 | >400 | >100 | 12.5 |
| | *Escherichia coli* | ATCC25922 | >400 | 50 | 1.56 |
| | | ATCC35218 | >400 | 25 | 1.56 |
| | *Serratia marcescens* | ATCC13880 | >400 | >100 | 100 |
| | *Klebsiella pneumoniae* | ATCC700603 | >400 | >100 | 50 |
| | *Haemophilus influenzae* | ATCC33391 | 50 | 6.25 | 0.78 |
| | | ATCC33533 | 100 | 3.13 | 0.39 |
| | | ATCC49247 | 50 | 6.25 | 25 |
| | | ATCC49766 | 25 | 6.25 | 0.78 |
| CAM: Clarithromycin, TC: Tetracycline | | | | | |

It is evident from the foregoing results that TFM shows selective antibacterial activities on causative organism of periodontal disease.

### Example 4

### (Preparation Example)

### 1. Tooth paste

The following components (parts by weight) were mixed together according to the ordinary procedure to prepare a tooth paste:

| | |
|---|---|
| Calcium hydrogen phosphate | 45.0 |
| Silicic acid anhydride | 2.0 |
| Carboxymethylcellulose | |
| sodium | 1.2 |
| Glycerin | 7.0 |
| Sorbitol | 15.0 |
| Saccharin sodium | 0.2 |
| Methylparaben | 0.2 |
| Sodium lauryl sulfate | 1.5 |
| Allantoin | 0.1 |
| chlorohydroxyaluminum | |
| Perfume | 0.9 |
| Trifluoromethionine | 0.1 |
| Purified water | balance |
| Total | 100.0 |

### 2. Tooth wash

The following components (parts by weight) were mixed together according to the ordinary procedure to prepare a tooth wash:

| | |
|---|---|
| Silicic acid anhydride | 7.5 |
| Carboxymethylcellulose sodium | 0.8 |
| Glycerin | 3.0 |
| Sorbitol | 30.0 |
| Saccharin sodium | 0.3 |
| Methylparaben | 0.1 |
| Sucrose fatty acid ester | 1.0 |
| Sodium fluoride | 0.2 |
| Trifluoromethionine | 0.1 |
| Purified water | balance |
| Total | 100.0 |

### 3. Mouth wash

The following components (parts by weight) were mixed together according to the ordinary procedure to prepare a mouth wash:

| | |
|---|---|
| Xylitol | 10.0 |
| Ethanol | 4.0 |
| Polyoxyethylene hardened castor oil | 0.2 |
| Myristic acid | 0.1 |
| L-arginine | 0.09 |
| Perfume | 0.15 |
| Coloring agent | trace amount |
| Trifluoromethionine | 0.1 |
| Purified water | balance |
| Total | 100.0 |

### INDUSTRIAL UTILITY

The present oral composition can effectively prevent and/or treat intraoral diseases such as bad breath, alveolar pyorrhea, periodontal disease, etc., and also the present oral composition can be daily used for prevention of bad breath, periodontal disease, etc. without killing intraoral non-pathogenic indigenous bacteria.

## Claims

1. An oral composition, which comprises trifluoromethionine as an effective component.

2. An oral composition according to Claim 1, which is a preparation for preventing and/or treating bad breath.

3. An oral composition according to Claim 1, which is a preparation for preventing and/or treating alveolar pyorrhea.

4. An oral composition according to Claim 1, which is a preparation for preventing and/or treating periodontal disease.
